# EUROPEAN PATENT APPLICATION

(11) **EP 2 123 262 A1**
(43) Date of publication of application: **25.11.2009**
(21) Application number: 08425351.7
(22) Date of filing: 20.05.2008
(51) Int. Cl.: A61K 9/50, A61K 9/51, A61K 31/198, A61P 25/28

(54) **Polyelectrolyte-encapsulated gold nanoparticles capable of crossing blood-brain barrier**

(71) Applicant: Consorzio per il Centro di Biomedica Moleculare Scrl, 34012 Basovizza (TS) (IT)
(72) Inventor:
(74) Representative: Spadaro, Marco

(57) **Abstract**

A gold-creatine nanoparticle is described, preferably covered with albumin or chitosan, together with a process for its preparation and its use as medicament, in particular for the treatment of stroke. Said gold nanoparticle is capable of crossing the blood-brain barrier.

## Description

### Technical field

The present invention refers to the medical field, in particular to pharmaceutical formulations for the selective release of drugs.

More in particular, the present invention refers to nanoparticles, to a process for their preparation and to their use as a medicament to treat diseases and conditions that require that the pharmaceutical agent, in particular creatine, is delivered through one or more physiological barriers, in particular the blood-brain barrier.

### State of the Art

One of the main tasks in the development of advanced pharmaceutical formulations is the production of delivery systems capable of providing a targeted release of drugs.

There is a need to ensure that these transport systems reach certain sites and should therefore be able to cross the physiological barriers, including the blood-brain barrier.

The nanoparticles are used for the controlled release and the delivery to specific sites of active pharmaceutical ingredients and possess a great potential in many areas of medicine.

There is also the need that said release systems are stable in physiological conditions.

Creatine is a guanidine compound endogenously produced by liver, kidney and pancreas (Juhn M.S., Tarnopolsky M., Oral creatine supplementation and athletic performance: a critical review. Clin J Sport Med 1998; 8:286 -297).

Creatine is known to increase muscle and brain phosphocreatine concentrations, and may inhibit the activation of the mitochondrial permeability transition, protects against neuronal degeneration in transgenic murine models of amyotrophic lateral sclerosis and Huntington's disease and in chemically mediated neurotoxicity (Tarnopolsky, M.A., et al., Potentiali for creatine and other therapies targeting cellular energy dysfunction in neurological disorders, Ann Neurol 2001; 49:561-574).

The need to get creatine in the cerebral stroke site still exists because the blood-brain barrier is one of the physiological barriers more difficult to cross.

There are pharmaceutical compositions and methods to increase the delivery of drugs and other agents through the blood-brain barrier (US 6,419,949, WO 89/11299, US 2002/115747, WO 02/69930, WO 2006/44660 and WO 2007/88066).

All these methods adopt different systems and no one refers to creatine.

An object of the invention is a system for transporting creatine through the blood-brain barrier that is capable of carrying a therapeutically effective dose and is stable under physiological conditions.

### Summary of the invention

It has now been found that gold nanoparticles are capable of delivering creatine through the blood-brain barrier, in particular when covered by an albumin layer.

Gold-creatine nanoparticles are an object of the present invention.

It is another object of the present invention a process for their preparation.

Further objects of the present invention are nanoparticles for use as a medicament, in particular for the treatment of stroke.

It is another object of the present invention a pharmaceutical composition comprising an effective amount of said nanoparticles.

These and other objects of the invention will be described in detail also by examples and figures.

### Description of the Invention

### Modes for Realizing the Invention

The present invention has been realized thanks to an accurate control of pH in the adsorption step of creatine on the surface of gold nanoparticles.

In a first preferred embodiment of the invention, the gold-creatine nanoparticle is covered by albumin.

Preferably, the gold core nanoparticle has a diameter ranging from 5 to 50 nm.

Once made, the nanoparticle according to the present invention has a diameter comprised between 100 and 200 nm.

Another object of the present invention is a process for the preparation of the nanoparticle, comprising adding a dispersion of gold core nanoparticles to a creatine solution at a pH of both said dispersion and said solution higher than 9, preferably at least 11.

According to another object of the present invention, the nanoparticle is here described any one of claims 1-4 for use as a medicament.

In a preferred embodiment of the invention, the nanoparticle is provided for use as a medicament for treating stroke, in particular neuroprotective in stroke.

In another of its aspects, the present invention provides a gold-creatine nanoparticle, said particle is covered with a polyelectrolyte capable of crossing the blood-brain barrier.

In a preferred embodiment of the invention, said polyelectrolyte is chitosan.

In all its embodiments, the present invention provides said nanoparticle for use a medicament, in particular as a system of drug delivery.

Therefore, it is another object of the present invention a pharmaceutical composition comprising an effective amount of nanoparticle as here described.

Pharmaceutical composition are known e can be prepared with conventional processes, for example as decribed in Remington's Pharmaceutical Sciences, last edition, Mack Publishing and Co.

The following example will further illustrate the invention.

Example

Materials and Methods:

### Materials

Deionized and filtered water (Milli-Q Academic, Millipore, France) was used in the preparation of all the suspensions and solutions. The chemicals used were purchased from Sigma-Aldrich (Germany) with analytical quality, and were not further purified. Gold nanoparticles were synthesized according to the citrate thermal reduction method developed by Turkevich et al. [J. Turkevich, P.C. Stevenson, J. Hilier, Trans. Faraday Soc. 11 (1951) 55]. Briefly, a gold sol was prepared by adding 4.5 mg of sodium tetrachloroaurate (III) dihydrate in 25 mL of Milli-Q grade water, and 1 mL of a 1 % sodium citrate tribasic dihydrate solution was rapidly added via a syringe into the boiling solution under vigorous stirring. The citrate ion acts both as reductant and stabilizer. It can clearly be seen how the color of the solution changes from yellowish to purple and finally red after the addition of the latter into the solution. After boiling for 20 minutes at 300 °C, the solution was left to cool at room temperature under moderate magnetic stirring. The result is a stable dispersion of gold particles with an average hydrodynamic diameter of about 20 nm (standard deviation of 10%). This value is in good agreement with the results reported by other authors elsewhere [J. Kunze, I. Burges, R. Nichols, C. Buess-Herman, J. Lipkowski, J. Elec. Chem., 599 (2007) 147-159].
Gold-creatine nanoparticles were synthesized by adding dropwise 1 mL of the previously obtained gold solution into a 0.5 mL solution of 2 mg/mL of creatine under moderate vortex rate. In order to have creatine molecules with a single charge, and for avoiding aggregation between the gold nanoparticles, the pH of both solutions was previously adjusted to 11.2. As result, the color of the solution remains red-pink, as it was originally. We noticed that, when the pH of both solutions is less than 11, the adsorption of creatine on the gold-nanoparticles surface is negligible, and the color of the solution turns to violet-blue, good indication that aggregation between the gold particles occurred. Finally, with the aim of improving the stability of the particles, an out-layer of albumin is incorporated. The albumin-covered creatine-gold particles were obtained adding 0.5 mL of the creatine-covered gold particles solution dropwise into 1 mL of 1 mg/mL albumin solution at pH 10. A drastic decrease of pH in the gold-creatine solution always shows a change in color, and the presence of aggregates. However, once the adsorption of albumin takes place, the system is stable, and the pH can be changed to physiological conditions.

### Electrical surface characterization

Electrophoretic mobility (uₑ) measurements were performed in a Malvern Zetasizer 2000 apparatus (Malvern Instruments, England) at room temperature. Measurements were carried out 24 hours after the preparation of the suspensions, and the pH was readjusted immediately before the mobility was measured.

### Determination of the concentration of creatine adsorbed

Adsorption of creatine from the nanogold solution was studied by HPLC method with an Agilent 1100 HPLC system (Agilent Technologies, Palo Alto, CA) equipped with a diode array detector, and a phase reverse column Atlantis T3 C18 (4.6 × 150 mm, 5 µm) from Waters (Waters Corp., Milford, USA). The samples were manually injected through a Rheodyne 7725i valve (Rheodyne, Cotati, USA) with a 50 µL loop.
The mobile phase was a phosphate buffer 10 mM, pH 5, with 5mM 1-pentanesulfonic acid (Fluka) as ion pairing agent (coded as buffer A). All the analysis of creatine were performed isocratically at a flow rate of 1 mL/min operating at a temperature of 30 °C. The volume injected was 10 µL. The eluate was simultaneously monitored for 10 minutes after the injection.

### Animal Model

Three C57 black female mice were treated intravenously (100µl) as following:
- Mouse n°1: control (not treated).
- Mouse n°2: negative control (mouse treated EV with 5nM of cyanine 5.5).
- Mouse n°3: experimental mouse (mouse treated with nanogold particles, coated with creatine and cyanine 5.5-labelled albumin, creatine: 50mg bound to the particles).
After the treatment mice were anesthetized using zolazepam plus xylazine (3.2µl/gr intramuscular) and shaved in the belly and in the skull.

Fluorescence microscopy

Images from the three animal models were acquired at the NIR wavelength of 670nm of excitation and 700nm of emission. The laser power, integration time and scan step were optimized for each scan.
For all the animals images were acquired for both the abdomen (mouse upside up) and for the skull (mouse upside down) to distinguish the auto-fluorescence in the control animal with respect to the fluorescence emitted from the treated ones. The abdomen scans allow to have informations about the biodistribution of the probes in the whole body and their pharmacokinetics (liver metabolization and bladder excretion), while the skull scans allow to assess the capability of the tested compound to cross the blood-brain barrier.
Scans were acquired at time zero, 1 hour, 2 hours , 2 hours and 30 minutes, 3 hours, 3 hours and 30 minutes, 4 hours, 19, 24 and 48 hours. Two days after the treatments, mice were sacrificed by cervical dislocation, the brains were explanted, washed in PBS and tissue fluorescence was analyzed ex vivo.

Results:

Effect of the pH

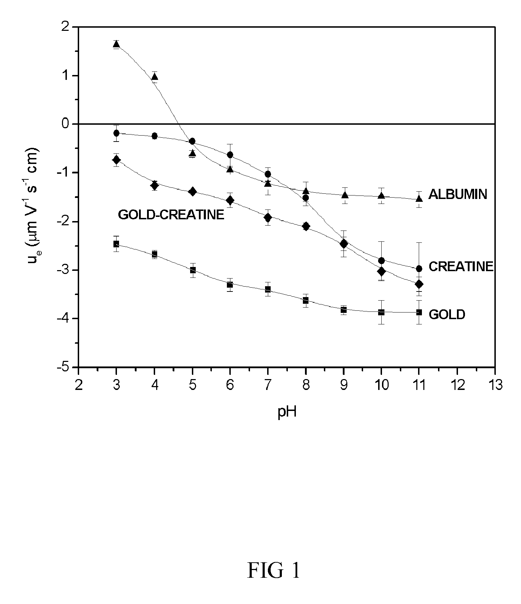

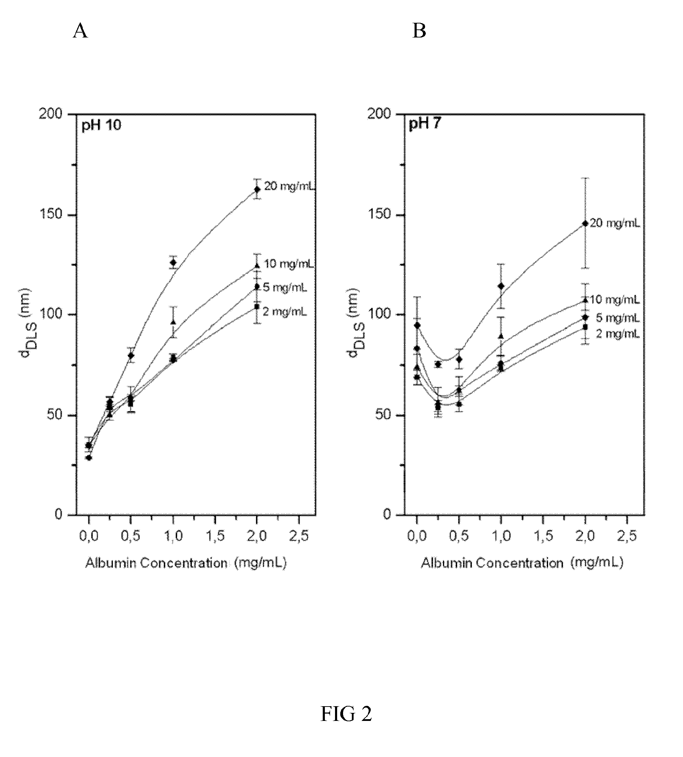

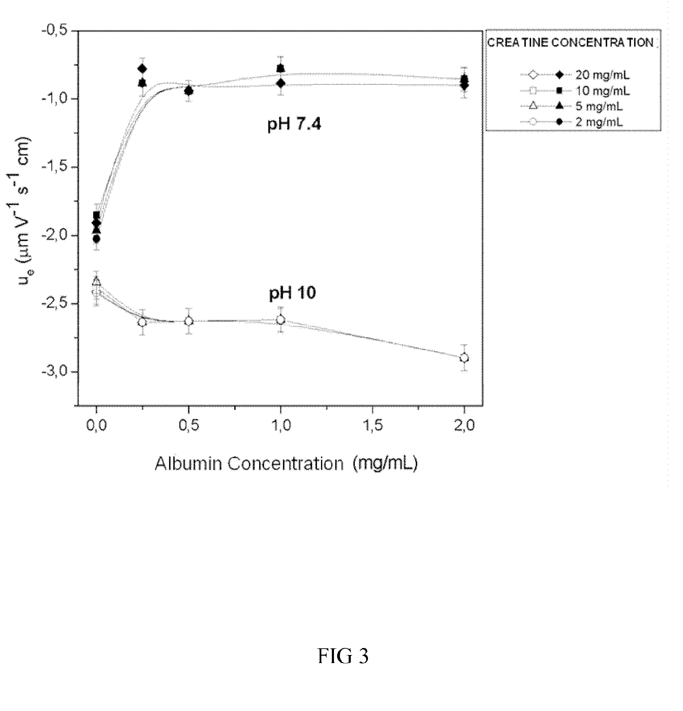

In Fig. 1. electrophoretic mobility as a function of the pH of the gold nanoparticles, creatine, gold-creatine composite particles, and albumin are shown. As observed, the gold nanoparticles present a negative electrophoretic mobility (uₑ) over the whole range of pH, characteristic of the citrate molecules adsorbed on them, becoming more negative with pH increase. Creatine electrophoretic mobility is also negative in the whole range of pH, although uₑ is very close to zero under acidic conditions. On the contrary, albumin molecules present an isoelectric point between pH 4.5-5, becoming more negative with pH increase.
This electrokinetic technique is a very useful tool for qualitatively checking the coating efficiency. The electrophoretic mobility of the creatine-covered gold composite particles is negative for the whole range of pH studied, it decreases as the pH becomes more basic and, as it can be seen, from an electrokinetic point of view, the results are qualitatively similar to the values obtained for the creatine molecules specially at basic pHs.
The adsorption of creatine by the gold particles, as well as albumin as outside layer has also been followed by dynamic light scattering measurements. Fig. 2 shows the average hydrodynamic diameter of the composite particles as a function of the concentration of albumin, for different concentrations of creatine at pH 10 (Fig. 2A) and at pH 7.4 (Fig. 2B). It is worthy to mention that, at pH 10, the measured diameter for the gold nanoparticles is (21±3) nm, and a good indication of the adsorption of creatine on their surface is the increase of the diameter size to (31±4) nm. The instability mentioned above of the creatine-covered gold composite particles can be clearly appreciated by comparing the size of the particles at pH 10 and at pH 7. As it can be seen, when the pH is decreased to 7.4, the size of the particles increases, the larger it is, the higher is the concentration of creatine (i.e. for concentrations of creatine of 20 mg/mL, the hydrodynamic diameter at pH 10: d_{pH10} ≈ 30 nm, increases to d_{pH7.4} ≈ 90 nm, when pH is 7.4). This increase in size comes together with a change in the color of the solution, as it was mentioned before. The adsorption of albumin can also be tracked by comparing the size of the particles: there is a significant enlargement on the size of the particles, as the concentration of albumin is increased. As in the case of the gold-creatine, uₑ measurements were performed on the albumin-covered creatine-gold composite particles. Fig. 3 shows the dependence of the electrophoretic mobility of the creatine-covered gold particles as a function of the concentration of albumin for different concentrations of creatine, for pH 10 and pH 7.4. As it can be seen: (i) in the absence of albumin, the uₑ of the creatine-gold particles do not present significant differences for the concentrations of creatine studied; as in Fig. 1, the more basic is the pH, the more negative is the uₑ; (ii) absorption of albumin by creatine-covered particles is clearly shown by a significant difference in the electrophoretic mobility; as it can be seen, at pH 10 there is a slight decrease of uₑ compared to the ones obtained in the absence of the albumin; on the contrary, at pH 7.4, the lower charge of the albumin showed in Fig. 1, contributes to screen the surface charge of the creatine-covered particles, compared to the one obtained without albumin; (iii) in any case, for both pH conditions, the effect of the concentration of albumin is also very little, as we have observed for the concentration of creatine. These results point out the clear protecting effect exerted by the protein when it covers the creatine-covered gold particles.

In vivo Distribution

It is noted that fluorescence lifetime increases as a function of time in the brain region, indicating the presence of the particles inside the brain.
The analysis with microscope shows that the particles according to the present invention are able to cross the blood-brain membrane.

Potential Application

Industrial Application
The present invention relates to the medical field, in particular to pharmaceutical formulations for the selective release of drugs that can cross the blood-brain barrier and reach the sites affected by brain stroke. Given the neuroprotective function of creatine, these pharmaceutical formulations may have an application in the treatment of neurodegenerative diseases such as Alzheimer's disease, Parkinson's disease and multiple sclerosis.

## Claims

1. Gold-creatine nanoparticle.

2. A nanoparticle according to claim 1 which is albumin-covered.

3. A nanoparticle according to claim 1 or 2, wherein the gold core nanoparticle has a diameter ranging from 5 to 50 nm.

4. A nanoparticle according to any one of claims 1-3, wherein the diameter of said nanoparticle ranges from 100 to 200 nm.

5. A process for the preparation of the nanoparticle of claim 1 comprising adding a dispersion of gold core nanoparticles to a creatine solution at a pH of both said dispersion and said solution higher than 9.

6. A process according to claim 5, wherein said gold core nanoparticles have a diameter ranging from 5 to 50 nm.

7. A process according to claim 5 or 6, wherein said gold nanoparticle has a diameter ranging from 100 to 200 nm.

8. Nanoparticle of any one of claims 1-4 for use as medicament.

9. Nanoparticle of claim 8 for use as medicament for the treatment of stroke.

10. Nanoparticle of claim 8 for use as neuroprotective in stroke.

11. Creatine-gold nanoparticle covered with a polyelectrolyte capable of crossing the blood-brain barrier.

12. Nanoparticle according to claim 11, wherein said polyelectrolyte is chitosan.

13. Nanoparticle of any one of claims 11-12 for use as medicament.

14. Nanoparticle of any one of claims 11-12 for use as drug-delivery system.

15. Pharmaceutical composition comprising an effective amount of nanoparticle of any one of claims 1-4 or of any one of claims 10-14.
